# EUROPÄISCHE PATENTSCHRIFT

(11) **EP 0 373 464 B1**
(45) Veröffentlichungstag und Bekanntmachung des Hinweises auf die Patenterteilung: **01.02.1995**
(21) Anmeldenummer: 89122285.3
(22) Anmeldetag: 02.12.1989
(51) Int. Cl.: C07D 213/26, C07D 213/61

(54) **Verfahren zur Herstellung von 2-Chlor-5-chlormethylpyridin**
Process for the preparation of 2-chloro-5-chloromethyl pyridine
Procédé de préparation de pyridine-2-chloro-5-chlorométhyle

(30) Priorität: 16.12.1988 DE 3842359
(43) Veröffentlichungstag der Anmeldung: 20.06.1990
(73) Patentinhaber: BAYER AG, 51368 Leverkusen (DE)
(72) Erfinder: Jelich, Klaus, Dr., D-5600 Wuppertal 1 (DE)

(56) Entgegenhaltungen:
- EP-A- 0 163 855
- EP-A- 0 281 965
- US-A- 2 695 902
- TETRAHEDRON LETTERS, Band 50, Nr. 49, November 1984, Seiten 5693-5696, Pergamon Press, Ltd, Oxford, GB; R.S. DAINTER et al.: "Abnormal nucleophilic substitution of 3-trichloromethylpyridines by methoxide"
- JOURNAL OF THE CHEMICAL SOCIETY, PERKIN TRANSACTIONS I, Februar 1989, Seiten 283-287, Cambridge, GB; R.S. DAINTER et al.: "Transformations of trichloromethyl groups during reactions of 3-trichloromethylpyridines with methoxide"
- JOURNAL OF HETEROCYCLIC CHEMISTRY, Band 10, Oktober 1973, Seiten 779-784, Provo; S. NESNOW et al.: "Pyridine nucleosides related to 5-fluorouracil (1)"

## Beschreibung

Die Erfindung betrifft ein neues Verfahren zur Herstellung von 2-Chlor-5-chlormethylpyridin, welches als Zwischenprodukt zur Herstellung von bekannten Insektiziden verwendet wird.

Es ist bekannt, daß man 2-Chlor-5-chlormethylpyridin in einem aufwendigen mehrstufigen Verfahren erhält, wenn man 2-Chlorpyridin-5-carbonsäure mit Thionylchlorid in das entsprechende Säurechlorid überführt, dieses gegebenenfalls mit Ethanol verestert, anschließend mit Natriumboranat zu Hydroxymethylverbindung reduziert und schließlich mit Thionylchlorid in der Seitenkette die Hydroxygruppe durch Chlor substituiert (vgl. z. B. US-PS 4 576 629; J. Org. Chem. 34, 3545 [1969]; J. Heterocycl. Chem. 16, 333 - 337 [1979]).

Nachteilig bei diesem Verfahren und prohibitiv für eine großtechnische Durchführbarkeit ist jedoch der hohe Preis der Ausgangsverbindung 2-Chlorpyridin-5-carbonsäure und des Reduktionsmittels Natriumboranat welches außerdem auch unter dem Aspekt der Wasserstofffreisetzung im Verlauf der Reaktion ein sicherheitstechnisches Problem darstellt.

Weiterhin ist bekannt, daß man 2-Chlor-5-chlormethylpyridin erhält, wenn man 2-Chlor-5-methylpyridin mit elementarem Chlor umsetzt (vgl. z. B. DE-A 36 30 046). Nachteilig bei diesem Verfahren ist jedoch, daß die Reaktion nicht einheitlich verläuft, so daß es nötig ist, um die Bildung von größeren Mengen an mehrfach chlorierten Nebenprodukten zu vermeiden, die Chlorierung frühzeitig abzubrechen, noch bevor die Umsetzung vollständig erfolgen konnte (vgl. auch EP-A 9 212; EP-A 65 358). Die entstehenden Gemische sind nur schwierig aufzutrennen und liefern Produkte mit nicht befriedigender Reinheit.

Es wurde nun gefunden, daß man 2-Chlor-5-chlormethylpyridin der Formel (I) 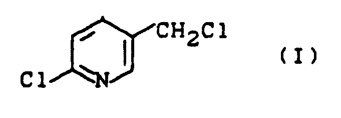
in hoher Ausbeute und großer Reinheit erhält, wenn man zunächst in einer 1. Stufe Nicotinsäure der Formel (II) 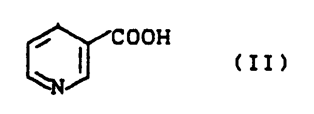
mit Phosphorpentachlorid in Gegenwart von Thionylchlorid und gegebenenfalls in Gegenwart eines Verdünnungmittels umsetzt,
dann in einer 2. Stufe das so erhältliche 3-Trichlormethylpyridin der Formel (III) 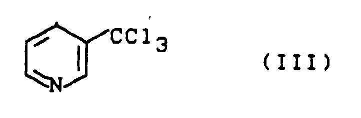
mit Alkalimetallalkoholaten der Formel (IV),

R - O - M (IV)

in welcher
- R: für Alkyl steht und
- M: für eine Alkalimetallkation steht,
gegebenenfalls in Gegenwart eines Verdünnungsmittels umsetzt,
dann in einer 3. Stufe die so erhältlichen Pyridinether-acetale der Formel (V), 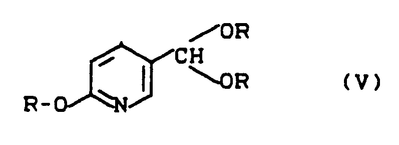
in welcher
- R: die oben angegebene Bedeutung hat,
mit Wasser gegebenenfalls in Gegenwart einer Katalysatorsäure umsetzt,
dann in der 4. Stufe die so erhältlichen Pyridinaldehyde der Formel (VI), 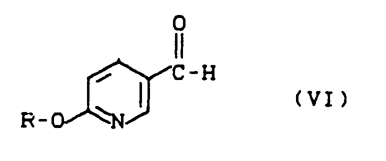
in welcher
- R: die oben angegebene Bedeutung hat,
mit molekularem Wasserstoff in Gegenwart eines Hydrierkatalysators und gegebenenfalls in Gegenwart eines Verdünnungsmittels hydriert
und schließlich in einer 5. Stufe die so erhältlichen Pyridylmethanole der Formel (VII), 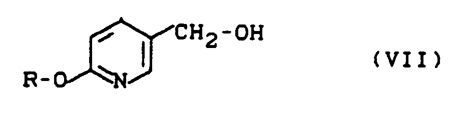
in welcher
- R: die oben angegebene Bedeutung hat,
mit einem Chlorierungsmittel gegebenenfalls in Gegenwart eines Verdünnungsmittels und gegebenenfalls in Gegenwart eines Reaktionshilfsmittels umsetzt,
wobei auch jeweils die zweite und die dritte Stufe oder die dritte und die vierte Stufe oder die zweite, die dritte und die vierte Stufe ohne Isolierung der Zwischenprodukte direkt in einem Reaktionsschritt durchgeführt werden können (sog. "Eintopfreaktionen").

An der erfindungsgemäßen Reaktionsabfolge sind eine Reihe von Gesichtspunkten als völlig überraschend und für den Fachmann nicht vorhersehbar einzustufen. Weiterhin ist durch die Kombination der 5 Reaktionsstufen das Verfahren in seiner Gesamtheit neu und erfinderisch.

So konnte es beispielsweise nicht erwartet werden, daß die Umsetzung der Nicotinsäure mit Phosphorpentachlorid in Gegenwart von Thionylchlorid gemäß der ersten Stufe des erfindungsgemäßen Verfahrens in glatter Reaktion und sehr hohen Ausbeuten zu dem gewünschten 3-Trichlormethylpyridin der Formel (III) führen würde, da einerseits aus dem Stand der Technik bekannt war, daß zur Erzielung einer höheren Ausbeute sehr reaktionsfähige, aufwendig herzustellende und schwierig zu handhabende Phenylphosphinchloride als Chlorierungsmittel bzw. Reaktionshilfsmittel erforderlich seien (vgl. hierzu US-PS 4 634 771) und da andererseits eigene Untersuchungen ergeben hatten, daß die einfache direkte Umsetzung von Nicotinsäure mit Phosphorpentachlorid (vgl. hierzu Tetrahedron Letters 25, 5693 - 5696 [1984]) in Substanz oder auch in Gegenwart von Verdünnungsmitteln nur Ausbeuten von maximal 5 % an gewünschter Trichlormethylpyridinverbindung der Formel (III) liefern.

Ebenfalls nicht vorhersehbar war die Tatsache, daß die Umsetzung von 2-Alkoxy-5-pyridylmethanolen der Formel (VII) mit Chlorierungsmitteln wie beispielsweise Phosphoroxychlorid oder Phosgen gemäß der fünften Stufe des erfindungsgemäßen Verfahrens zu einem gleichzeitigen Austausch sowohl der Hydroxygruppe als auch der Alkoxygruppe gegen jeweils einen Chlorrest führen würde, da einerseits aus dem Stand der Technik bekannt war, daß entweder für eine großtechnische Durchführung wenig geeignete "Vilsmeier-Haack-Bedingungen" (d. h. Phosphoroxychlorid in Gegenwart von großen Mengen Dimethylformamid, welches bei der Aufarbeitung erhebliche Abwassermengen beschert) für eine Überführung von 2-Methoxypyridin in 2-Chlorpyridin erforderlich seien, wobei Phosphoroxychlorid in Abwesenheit von Dimethylformamid keinerlei Umsetzung bewirkte. Außerdem liefert diese Reaktion mit weniger als 40 % Ausbeute nur ein äußerst unsauberes Produkt, welches aufwendig chromatographisch gereinigt werden muß (vgl. hierzu Synthesis 1984, 743 - 745). Andererseits war bekannt, daß auch vergleichbare Umsetzungen mit Thionylchlorid ausschließlich einen Austausch an der Hydroxygruppe bewirken, wobei die Etherfunktion unverändert bleibt (vgl. hierzu EP 163 855).

Schließlich war es auch nicht vorhersehbar, daß es möglich sein würde, jeweils die zweite und die dritte Stufe des erfindungsgemäßen Verfahrens oder die dritte und die vierte Stufe des erfindungsgemäßen Verfahrens oder die zweite und die dritte und die vierte Stufe des erfindungsgemäßen Verfahrens ohne Isolierung der Zwischenprodukte direkt durchzuführen, da solche "Eintopfreaktionen" insbesondere wenn sie sich über mehr als zwei Reaktionsschritte erstrecken in der Regel zu empfindlichen Ausbeuteverlusten führen und/oder aufwendige Reinigungsvorgänge erforderlich machen und nur in Ausnahmefällen zu befriedigenden Resultaten führen.

Als besonderer Vorteil der erfindungsgemäßen Reaktionssequenz muß herausgestellt werden, daß das Ausgangsprodukt Nicotinsäure ein billiges, großtechnisch herstellbares Ausgangsmaterial darstellt; ferner daß alle Umsetzungen mit leicht zugänglichen Reagenzien, unter technisch leicht herstellbaren Reaktionsbedingungen selektiv und in hoher Ausbeute durchführbar sind und daß nicht zuletzt insbesondere die oben angesprochenen Varianten ohne Isolierung bestimmter Zwischenstufen zu einer rationellen und ökonomischen Synthese der gewünschten Zielverbindung führen.

Verwendet man beispielsweise Nicotinsäure als Ausgangsverbindung, Thionylchlorid und Phosphorpentachlorid als Reaktionspartner in der ersten Stufe, Natriummethylat als Reagenz in der zweiten Stufe, verdünnte Salzsäure als Reaktionshilfsmittel in der dritten Stufe, Palladium auf Aktivkohle als Hydrierkatalysator in der vierten Stufe und Phosgen in Gegenwart von Dibutylformamid als Chlorierungsmittel in der fünften Stufe, so läßt sich der Reaktionsablauf des erfindungsgemäßen Verfahrens durch das folgende Formelschema darstellen:
1. Stufe: 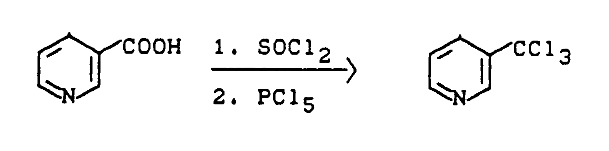
2. Stufe: 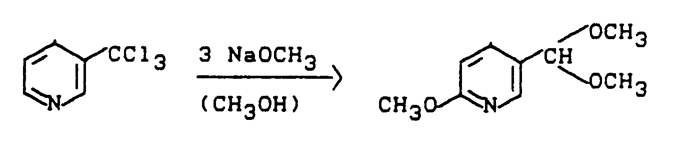
3. Stufe: 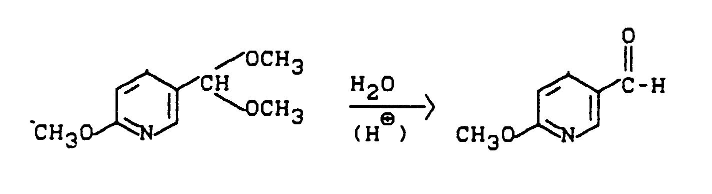
4. Stufe: 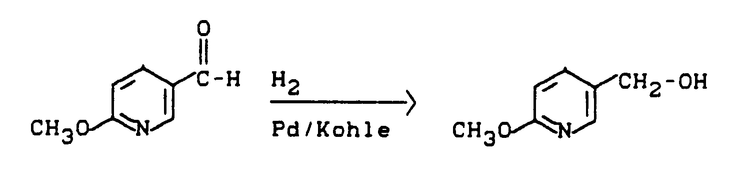
5. Stufe: 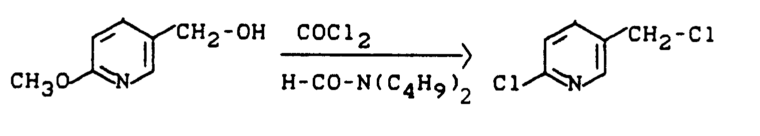
Als Verdünnungsmittel zur Durchführung der ersten Stufe des erfindungsgemäßen Verfahrens kommen inerte organische Lösungsmittel in Frage. Mit besonderem Vorzug verwendet man Benzol, Toluol, Xylol, Chlorbenzol, Dichlorbenzol, Nitrobenzol oder Phosphoroxychlorid. Es ist auch möglich, die erste Stufe des erfindungsgemäßen Verfahrens ohne Verwendung eines Verdünnungsmittels direkt in Substanz durchzuführen.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen 80 °C und 180 °C, vorzugsweise bei Temperaturen zwischen 110 °C und 160 °C.

Zur Durchführung der ersten Stufe des erfindungsgemäßen Verfahrens setzt man pro Mol an Nicotinsäure der Formel (II) entweder 2 bis 4 Mol an Phosphorpentachlorid oder zur Vermeidung eines größeren Phosphorpentachloridüberschusses zunächst 1 bis 2 Mol Thionylchlorid (hierbei wird das Ausgangsprodukt Nicotinsäure in das Hydrochlorid des entsprechenden Säurechlorids überführt) und anschließend 1 bis 2 Mol an Phosphorpentachlorid ein. Es ist auch möglich, das Phosphorpentachlorid aus einer entsprechenden Menge Phosphortrichlorid und einer equivalenten Menge Chlor direkt im Reaktionsgefäß herzustellen. Im Verlauf der Reaktion erweist es sich als vorteilhaft, freiwerdendes Phosphoroxychlorid kontinuierlich abzudestillieren.

Die Isolierung des Reaktionsproduktes der Formel (III) kann durch Destillation erfolgen; man kann aber auch das Rohprodukt für die weitere Umsetzung einsetzen.

Die zur Durchführung der zweiten Stufe des erfindungsgemäßen Verfahrens als Ausgangsstoffe benötigten Alkalimetallalkoholate sind durch die Formel (IV) allgemein definiert. In dieser Formel (IV) steht R vorzugsweise für geradkettiges oder verzweigtes Alkyl mit 1 bis 4 Kohlenstoffatomen, insbesondere für Methyl, Ethyl, Isopropyl, i-Butyl oder sec-Butyl.

M steht vorzugsweise für ein Natrium- oder Kaliumkation, insbesondere für ein Natriumkation.

Die Alkalimetallalkoholate der Formel (IV) sind allgemein bekannte Verbindungen; sie können gegebenenfalls in situ aus Alkalimetallhydroxiden und entsprechenden Alkoholaten erzeugt werden.

Als Verdünnungsmittel zur Durchführung der zweiten Stufe des erfindungsgemäßen Verfahrens kommen ebenfalls inerte organische Lösungsmittel in Frage. Mit besonderem Vorzug verwendet man niedere Alkylalkohole, welche den gleichen Alkylrest tragen, durch den die als Reaktionspartner verwendeten Alkalimetallalkoholate der Formel (IV) gekennzeichnet sind, insbesondere Methanol, Ethanol, Isopropanol oder Isobutanol.

Die Reaktionstemperaturen können bei der Durchführung der zweiten Stufe des erfindungsgemäßen Verfahrens in einem größeren Bereich variiert werden. Im allgemeinen arbeitet man bei Temperaturen zwischen 0 °C und 120 °C, vorzugsweise bei Temperaturen zwischen 20 °C und 90 °C.

Zur Durchführung der zweiten Stufe des erfindungsgemäßen Verfahrens setzt man pro Mol an 3-Trichlormethylpyridin der Formel (III) im allgemeinen 3.0 bis 15.0 Mol, vorzugsweise 3.0 bis 6.0 Mol an Alkalimetallalkoholat der Formel (IV) ein.
Die Reaktionsdurchführung, Aufarbeitung und Isolierung der Reaktionsprodukte erfolgt nach bekannten Methoden (vgl. die Herstellungsbeispiele).

Als Katalysatorsäure zur Durchführung der dritten Stufe des erfindungsgemäßen Verfahrens kommen verdünnte anorganische oder organische Säuren in Frage. Vorzugsweise verwendet man verdünnte wäßrige Salzsäure, Schwefelsäure, Ameisensäure oder Essigsäure als Reaktionsmedium. Es ist auch möglich, die Reaktion ohne Einsatz einer Katalysatorsäure in reinem Wasser als Reaktionsmedium durchzuführen.

Die Reaktionstemperaturen können bei der Durchführung der dritten Stufe des erfindungsgemäßen Verfahrens in einem größeren Bereich variiert werden. Im allgemeinen arbeitet man bei Temperaturen zwischen 0 °C und 100 °C, vorzugsweise bei Temperaturen zwischen 20 °C und 80 °C.

Zur Durchführung der dritten Stufe des erfindungsgemäßen Verfahrens setzt man pro Mol an Pyridin-ether-acetal der Formel (V) im allgemeinen 10.0 bis 50.0 Mol an Wasser und gegebenenfalls 0.01 bis 10.0 Mol an Katalysatorsäure ein.
Die Reaktionsdurchführung, Aufarbeitung und Isolierung der Reaktionsprodukte erfolgt nach allgemein üblichen Methoden (vgl. auch die Herstellungsbeispiele).

Als Hydrierkatalysatoren zur Durchführung der 4. Stufe des erfindungsgemäßen Verfahrens kommen übliche Edelmetall-, Edelmetalloxid- oder Raney-Katalysatoren, gegebenenfalls auf einem geeigneten Träger, wie beispielsweise Aktivkohle, Aluminiumoxid oder Siliciumdioxid in Frage. Mit besonderem Vorteil verwendet man Palladium auf Aktivkohle oder Raney-Nickel.

Als Verdünnungsmittel zur Durchführung der vierten Stufe des erfindungsgemäßen Verfahrens kommen inerte organische Lösungsmittel in Frage. Hierzu gehören insbesondere Ether wie Diethylether, Dioxan, Tetrahydrofuran oder Ethylglykoldimethyl- oder -diethylether, Alkohole wie Methanol, Ethanol, Propanol, Butanol, Ethylenglykolmonomethylether oder Ethylenglykolmonoethylether oder Säuren wie beispielsweise Essigsäure.

Die Reaktionstemperaturen können bei der Durchführung der vierten Stufe des erfindungsgemäßen Verfahrens in einem größeren Bereich variiert werden. Im allgemeinen arbeitet man bei Temperaturen zwischen 0 °C und 150 °C, vorzugsweise bei Temperaturen zwischen 20 °C und 100 °C.

Die vierte Stufe des erfindungsgemäßen Verfahrens wird entweder unter Normaldruck oder unter erhöhtem oder vermindertem Druck durchgeführt. Im allgemeinen arbeitet man in Druckbereichen zwischen 0,01 und 200 bar, vorzugsweise zwischen 0,1 und 100 bar.

Zur Durchführung der vierten Stufe des erfindungsgemäßen Verfahrens setzt man pro Mol an Pyridinaldehyd der Formel (VI) im allgemeinen 1.0 bis 20.0 Mol, vorzugsweise 1.0 bis 5.0 Mol an Wasserstoff und 0.0001 bis 1.0 Mol, vorzugsweise 0.01 bis 0.1 Mol an Hydrierkatalysator ein.
Die Reaktionsdurchführung, Aufarbeitung und Isolierung der Reaktionsprodukte erfolgt nach allgemein üblichen Methoden (vgl. auch die Herstellungsbeispiele).

Als Chlorierungsmittel zu Durchführung der fünften Stufe des erfindungsgemäßen Verfahren kommen insbesondere Phosphorpentachlorid, Phsophoroxychlorid oder Phosgen, sowie auch Gemische dieser Verbindungen in Frage.

Die fünfte Stufe des erfindungsgemäßen Verfahrens kann entweder ohne Zusatz eines Verdünnungmittels direkt in Substanz oder in Gegenwart eines geeigneten Verdünnungsmittels duchgeführt werden. Hierzu gehören insbesondere aliphatische, alicyclische oder aromatische, gegebenenfalls halogenierte Kohlenwasserstoffe, wie beispielsweise Benzol, Toluol, Xylol, Chlorbenzol oder Dichlorbenzol, Petrolether, Hexan, Cyclohexan, Dichlormethan, Chloroform oder Tetrachlorkohlenstoff.

Die fünfte Stufe des erfindungsgemäßen Verfahrens kann gegebenenfalls in Gegenwart eines geeigneten Reaktionshilfsmittels durchgeführt werden. Als solche kommen tertiäre Amine, wie Triethylamin, N,N-Dimethylanilin, Pyridin oder N,N-Dimethylaminopyridin in Frage, außerdem auch katalytische Mengen an Formamiden wie beispielsweise N,N-Dimethylformamid oder N,N-Dibutylformamid oder anorganische Metallchloride wie Magnesiumchlorid oder Lithiumchlorid.

Die Reaktionstemperaturen können bei der Durchführung der fünften Stufe des erfindungsgemäßen Verfahrens in einem größeren Bereich variiert werden. Im allgemeinen arbeitet man bei Temperaturen zwischen 20 °C und 200 °C, vorzugsweise bei Temperaturen zwischen 60 °C und 150 °C.

Zur Durchführung der fünften Stufe des erfindungsgemäßen Verfahrens setzt man pro Mol an Pyridylmethanol der Formel (VII) im allgemeinen 1.0 bis 10.0 Mol, vorzugsweise 1.0 bis 5.0 Mol an Chlorierungsmittel und gegebenenfalls 0.01 bis 3.0 Mol, vorzugsweise 0.1 bis 2.0 Mol an Reaktionshilfsmittel ein.

Der Verlauf der Reaktion läßt sich insbesondere bei der Verwendung von gasförmigen Chlorierungsmitteln mit Hilfe von dünnschichtchromatographischer Kontrolle verfolgen. Die Aufarbeitung erfolgt nach üblichen Verfahren.

Bei der Durchführung der "Eintopfvarianten" geht man so vor, daß man das aus der 2. Stufe des erfindungsgemäßen Verfahrens erhältliche Reaktionsgemisch, welches gelöst in Methanol vorliegt*⁾, bei Raumtemperatur mit Wasser und gegebenenfalls mit einer entsprechenden Menge an Katalysatorsäure versetzt, dann bei der erforderlichen Temperatur 0,5 bis 20 Stunden rührt**⁾ und das so erhältliche Reaktionsgemisch mit Hydrierkatalysator und gegebenenfalls mit zusätzlichem Lösungsmittel versetzt, in üblicher Weise bei der erforderlichen Temperatur und dem erforderlichen Druck hydriert, nach Beendigung neutralisiert, unlösliche Bestandteile abfiltriert und das Filtrat destillativ aufarbeitet (vgl. auch die Herstellungsbeispiele).
*⁾ An dieser Stelle ist es auch möglich, isoliertes und gereinigtes Produkt aus der 2. Stufe des erfindungsgemäßen Verfahren einzusetzen.
**⁾ An dieser Stelle ist es auch möglich aufzuarbeiten, wie für die 3. erfindungsgemäße Stufe beschrieben und die anschließende Hydrierung in getrennter Reaktion durchzuführen.

Die mit Hilfe des erfindungsgemäßen Verfahrens erhältliche Verbindung 2-Chlor-5-chlormethylpyridin der Formel (I) ist eine bekannte Verbindung und kann beispielsweise als Zwischenprodukt für die Herstellung von insektiziden Nitromethylenverbindungen verwendet werden (vgl. z. B. EP-A 163 855; EP-A 192 060; EP-A 259 738; EP-A 254 859).

### Herstellungsbeispiele

### 1. Stufe:

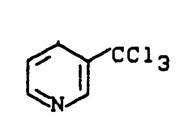

Zu 250 ml Thionylchlorid gibt man im Verlauf von 10 Minuten 123,1 g (1 Mol) Nicotinsäure, wobei sich die Mischung auf 50 °C erwärmt. Nach beendeter Zugabe rührt man 15 Minuten bei 55 °C und destilliert dann überschüssiges Thionylchloird unter vermindertem Druck ab. Danach gibt man 275 g (2 Mol) Phosphortrichlorid zu und leitet im Verlauf von 2 Stunden insgesamt 140 g (2 Mol) getrocknetes Chlorgas ein, wobei sich die Mischung auf 70 °C erwärmt. Danach erhitzt man für eine Stunde auf 150 °C, wobei freiwerdendes Phosphoroxychlorid kontinuierlich abdestilliert wird. Zur Aufarbeitung wird abgekühlt, 600 ml Essigester zugegeben, die Mischung in Eiswasser gegossen, durch portionsweise Zugabe von Natriumcarbonat unter Kühlen schwach alkalisch gestellt, die organische Phase abgetrennt und die wäßrige Phase mit 300 ml Essigester extrahiert. Die vereinigten organischen Phasen werden über Magnesiumsulfat getrocknet, im Vakuum eingeengt und im Wasserstrahlvakuum destilliert.

Man erhält 176,8 g (89 % der Theorie) an 3-Trichlormethylpyridin vom Siedepunkt 105 °C - 107 °C bei 15 mbar.

### 2. Stufe:

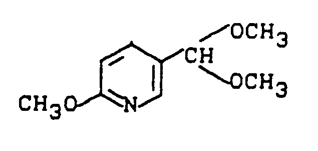

Zu 140,7 g (0,515 Mol) einer Lösung von Natriummethylat in Methanol gibt man bei Rückflußtemperatur tropfenweise unter Rühren im Verlauf von 45 Minuten 31,6 g (0,161 Mol) 3-Trichlormethylpyridin. Nach beendeter Zugabe rührt man weitere 3 Stunden bei Rückflußtemperatur, kühlt dann ab, filtriert, engt das Filtrat ein, nimmt den Rückstand in Dichlormethan auf, filtriert ein weiteres Mal, engt das Filtrat ein und destilliert den Rückstand im Vakuum.

Man erhält 24,5 g (83 % der Theorie) an 2-Methoxy-5-bis-(methoxy)methyl-pyridin vom Siedepunkt 54 °C - 55 °C bei 0,25 mbar.

### 3. Stufe:

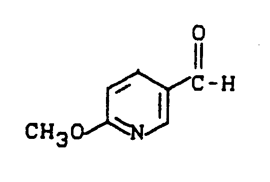

Zu 20 g (0,109 Mol) 2-Methoxy-5-bis-(methoxy)methyl-pyridin in 100 ml Wasser gibt man 20 ml konzentrierte wäßrige Salzsäure, rührt 1 Stunde bei Raumtemperatur, kühlt dann auf 0 °C und saugt den ausgefallenen Feststoff ab.

Man erhält 12,9 g (82 % der Theorie) an 6-Methoxypyridin-3-aldehyd vom Schmelzpunkt 47 °C - 49 °C.

### 4. Stufe:

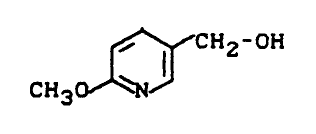

Zu 10,9 g (0,08 Mol) 6-Methoxypyridin-3-aldehyd in 100 ml Ethanol gibt man 2 g Raney-Nickel und hydriert die Mischung anschließend bei 75 °C und 50 bar Wasserstoffdruck 3 Stunden lang. Zur Aufarbeitung wird der Katalysator abfiltriert und das Filtrat im Vakuum eingeengt.

Man erhält 11,1 g (100 % der Theorie) an 2-Methoxy-5-hydroxymethylpyridin als Öl, welches destillativ gereinigt werden kann, vom Siedepunkt 85 °C - 88 °C bei 0,6 mbar.

### 5. Stufe:

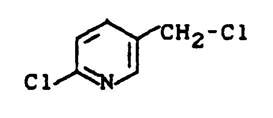

Zu 28,6 g (0,206 Mol) 2-Methoxy-5-hydroxymethylpyridin in 100 ml Toluol gibt man 6,5 g (0,041 Mol) N,N-Dibutylformamid, erhitzt auf 80 °C und leitet so lange einen langsamen Phosgenstrom in die Lösung, bis im Dünnschichtchromatogram (Kieselgel; Laufmittel Petrolether/Essigester 5 : 1) kein Ausgangsprodukt mehr nachweisbar ist (ca. 2,5 Stunden). Zur Aufarbeitung entfernt man überschüssiges Phosgen durch Ausblasen mit Stickstoff bei 80 °C, kühlt dann auf Raumtemperatur ab, gibt Wasser zu, stellt durch Zugabe von Natriumcarbonat einen leicht alkalischen pH-Wert ein, trennt die organische Phase ab, extrahiert die wäßrige Phase mit 50 ml Toluol, trocknet die vereinigten organischen Phasen über Magnesiumsulfat und entfernt das Lösungsmittel unter vermindertem Druck.

Durch Destillation im Hochvakuum erhält man 27,6 g (88 % der Theorie) an 2-Chlor-5-chlormethylpyridin vom Siedepunkt 70 °C - 80 °C bei 1 mn.

### 2. Stufe + 3. Stufe + 4. Stufe: (Eintopfversion)

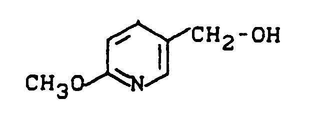

Zu 344 g (1,6 Mol) einer Lösung von Natriummethylat in Methanol gibt man bei Rückflußtemperatur tropfenweise unter Rühren im Verlauf von 25 Minuten 98,3 g (0,5 Mol) 3-Trichlormethylpyridin, erhitzt nach beendeter Zugabe für weitere 5 Stunden auf Siedetemperatur, kühlt dann auf Raumtemperatur ab, gibt 100 ml Wasser zu und stellt durch Zugabe von 15 ml konzentrierter wäßriger Salzsäure einen pH-Wert von 3 bis 4 ein. Das Gemisch wird 14 Stunden bei Raumtemperatur gerührt, dann mit 10 g Palladium auf Aktivkohle (10 %ig) versetzt, mit 40 ml Wasser versetzt und unter schnellem Rühren bei Normaldruck hydriert. Nach 3,5 Stunden (Wasserstoffaufnahme: 10,790 l) wird mit gesättigter wäßriger Natriumhydrogencarbonatlösung neutralisiert, filtriert, das Filtrat eingeengt, der Rückstand in Essigester aufgenommen, erneut filtriert, eingeengt und destilliert.

Man erhält 52,5 g (76,6 % der Theorie) an 2-Methoxy-5-hydroxymethylpyridin vom Siedepunkt 85 °C - 88 °C bei 0,6 mbar.

### 2. Stufe + 3. Stufe:

### (Eintopfversion)

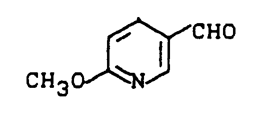

Zu einer Mischung aus 32,6 g (0,815 Mol) Natriumhydroxid und 200 ml Methanol werden bei Rückflußtemperatur 50 g (0,254 Mol) 3-Trichlormethyl-pyridin innerhalb von 15 Minuten tropfenweise gegeben. Das Reaktionsgemisch wird weitere 60 Minuten unter Rückfluß erhitzt, auf 20 °C abgekühlt und dann zunächst mit 100 ml Wasser und anschließend mit 25 ml konz. Salzsäure versetzt. Nach 3 Stunden Rühren bei 20 °C wird auf ca. 100 ml eingeengt, wieder mit Wasser verdünnt und zweimal mit Essigsäureethylester extrahiert. Die vereinigten Extraktionslösungen werden mit Natriumsulfat getrocknet und filtriert. Vom Filtrat wird das Lösungsmittel im Wasserstrahlvakuum sorgfältig abdestilliert.

Man erhält 27,2 g (78,5 % der Theorie) 6-Methoxypyridin-3-aldehyd vom Schmelzpunkt 47 °C - 49 °C.

## Patentansprüche

1. Verfahren zur Herstellung von 2-Chlor-5-chlormethylpyridin der Formel (I) 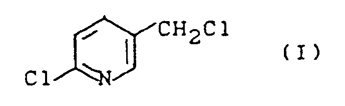 dadurch gekennzeichnet, daß man Nicotinsäure der Formel (II) 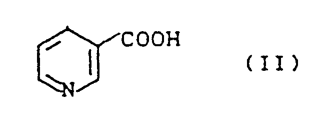 mit Phosphorpentachlorid in Gegenwartwart von Thionylchlorid und gegebenenfalls in Gegenwart eines inerten organischen Lösungsmittelsumsetzt.
dann in einer 2. Stufe das so erhältliche 3-Trichlormethylpyridin der Formel (III) 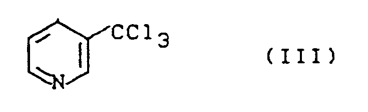 mit Alkalimetallalkoholaten der Formel (IV),
R - O - M (IV)
in welcher
R für Alkyl steht und
M für eine Alkalimetallkation steht,
gegebenenfalls in Gegenwart eines Verdünnungsmittels umsetzt,
dann in einer 3. Stufe die so erhältlichen Pyridinether-acetale der Formel (V), 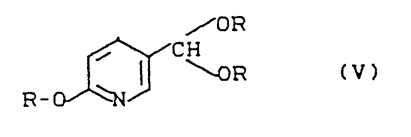 in welcher
R die oben angegebene Bedeutung hat,
mit Wasser gegebenenfalls in Gegenwart einer Katalysatorsäure umsetzt,
dann in der 4. Stufe die so erhältlichen Pyridinaldehyde der Formel (VI), 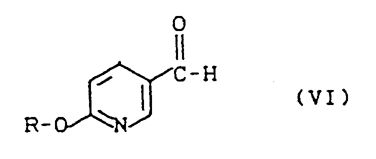 in welcher
R die oben angegebene Bedeutung hat,
mit molekularem Wasserstoff in Gegenwart eines Hydrierkatalysators und gegebenenfalls in Gegenwart eines Verdünnungsmittels hydriert
und schließlich in einer 5. Stufe die so erhältlichen Pyridylmethanole der Formel (VII), 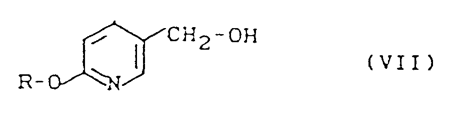 in welcher
R die oben angegebene Bedeutung hat,
mit einem Chlorierungsmittel gegebenenfalls in Gegenwart von aliphatischen, alicyclischen, aromatischen gegebenenfalls halogenierten Kohlenwasserstoffen als Verdünnungsmittel und in Gegenwart von tertiären Aminen, anorganischen Metallchloriden und katalytischen Mengen Formamiden umsetzt.

2. Verfahren gemäß Anspruch 1, dadurch gekennzeichnet, daß jeweils die zweite und die dritte Stufe oder die dritte und die vierte Stufe oder die zweite, dritte und vierte Stufe ohne Isolierung der Zwischenprodukte direkt in einem Reaktionsschritt durchgeführt werden.

3. Verfahren gemäß Anspruch 1 und 2, dadurch gekennzeichnet, daß die zweite und dritte Stufe ohne Isolierung der Zwischenprodukte durchgeführt werden.

4. Verfahren gemäß Anspruch 1 und 2, dadurch gekennzeichnet, daß die dritte und vierte Stufe ohne Isolierung der Zwischenprodukte durchgeführt werden.

5. Verfahren gemäß Anspruch 1 und 2, dadurch gekennzeichnet, daß die zweite, dritte und vierte Stufe ohne Isolierung der Zwischenprodukte durchgeführt werden.

## Claims

1. Process for the preparation of 2-chloro-5-chloromethylpyridine, of the formula (I), 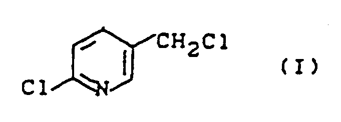 characterized in that nicotinic acid of the formula (II) 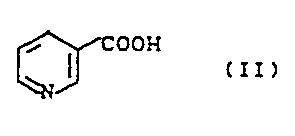 is reacted with phosphorus pentachloride in the presence of thionyl chloride and if appropriate in the presence of an inert organic solvent,
the resulting 3-trichloromethylpyridine, of the formula (III), 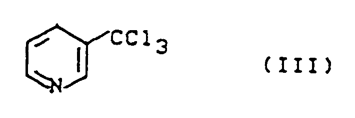 is then reacted, in a 2nd step, with alkali metal alkoxides of the formula (IV)
R - O - M (IV)
in which
R represents alkyl and
M represents an alkali metal cation,
if appropriate in the presence of a diluent,
the resulting pyridine ether acetals of the formula (V) 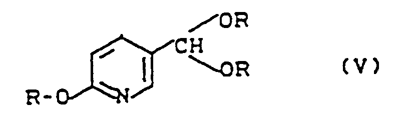 in which
R has the abovementioned meaning
are then reacted in a 3rd step with water, if appropriate in the presence of a catalyst acid,
the resulting pyridine aldehydes of the formula (VI) 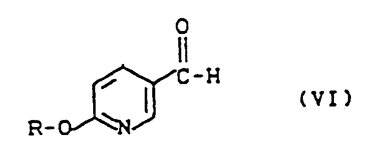 in which
R has the abovementioned meaning
are then hydrogenated in the 4th step with molecular hydrogen in the presence of a hydrogenation catalyst and, if appropriate, in the presence of a diluent,
and, finally, the resulting pyridylmethanols of the formula (VII) 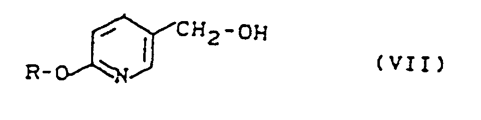 in which
R has the abovementioned meaning
are reacted in a 5th step with a chlorinating agent, if appropriate in the presence of aliphatic, alicyclic, aromatic, optionally halogenated hydrocarbons as diluent and in the presence of tertiary amines, inorganic metal chlorides and catalytic amounts of formamides.

2. Process according to Claim 1, characterized in that either step two and step three, or step three and step four, or steps two, three and four, are carried out directly in one reaction step without isolation of the intermediates.

3. Process according to Claim 1 and 2, characterized in that steps two and three are carried out without isolation of the intermediates.

4. Process according to Claim 1 and 2, characterized in that steps three and four are carried out without isolation of the intermediates.

5. Process according to Claim 1 and 2, characterized in that steps two, three and four are carried out without isolation of the intermediates.

## Revendications

1. Procédé de production de 2-chloro-5-chlorométhylpyridine de formule (I) 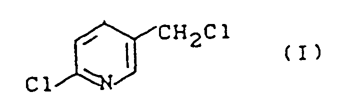 caractérisé en ce qu'on fait réagir de l'acide nicotinique de formule (II) 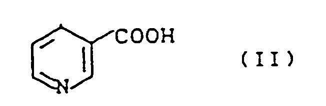 avec du pentachlorure de phosphore en présence de chlorure de thionyle et, le cas échéant, en présence d'un solvant organique inerte, puis, dans une deuxième étape, on fait réagir la 3-trichlorométhylpyridine ainsi obtenue de formule (III) 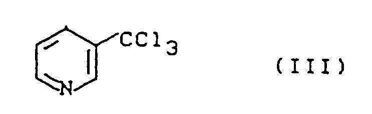 avec des alcoolates de métaux alcalins de formule (IV)
R - O - M (IV)
dans laquelle
R est un groupe alkyle et
M est un cation de métal alcalin,
le cas échéant en présence d'un diluant, après quoi dans une troisième étape, on fait réagir les acétals d'éther de pyridine ainsi obtenus de formule (V) 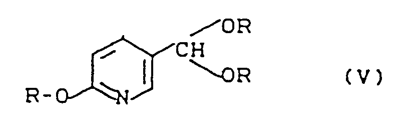 dans laquelle
R a la définition indiquée ci-dessus,
avec l'eau, le cas échéant en présence d'un acide catalyseur,
puis, dans la quatrième étape, on hydrogène les pyridinealdéhydes ainsi obtenus de formule (VI) 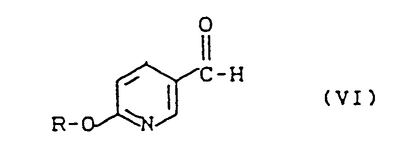 dans laquelle
R a la définition indiquée ci-dessus,
avec de l'hydrogène moléculaire en présence d'un catalyseur d'hydrogénation et en la présence éventuelle d'un diluant, et finalement, dans une cinquième étape, on fait réagir les pyridylméthanols ainsi obtenus de formule (VII) 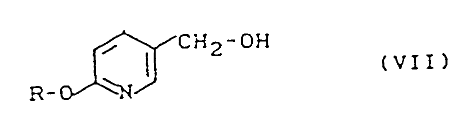 dans laquelle
R a la définition indiquée ci-dessus,
avec un agent de chloration, le cas échéant en présence d'hydrocarbures aliphatiques, alicycliques, aromatiques, éventuellement halogénés comme diluants et en présence d'amines tertiaires, de chlorures métalliques inorganiques et de quantités catalytiques de formamides.

2. Procédé suivant la revendication 1, caractérisé en ce qu'on conduit chacune des deuxième et troisième étapes ou des troisième et quatrième étapes ou des deuxième, troisième et quatrième étapes directement en une seule et même phase réactionnelle, sans isolement des produits intermédiaires.

3. Procédé suivant les revendications 1 et 2,caractérisé en ce que les deuxième et troisième étapes sont conduites sans isolement des produits intermédiaires.

4. Procédé suivant les revendications 1 et 2, caractérisé en ce que les troisième et quatrième étapes sont conduites sans isolement des produits intermédiaires.

5. Procédé suivant les revendications 1 et 2, caractérisé en ce que les deuxième, troisième et quatrième étapes sont conduites sans isolement des produits intermédiaires.
